# EUROPÄISCHE PATENTSCHRIFT

(11) **EP 2 242 420 B1**
(45) Veröffentlichungstag und Bekanntmachung des Hinweises auf die Patenterteilung: **28.02.2018**
(21) Anmeldenummer: 09700823.9
(22) Anmeldetag: 09.01.2009
(51) Int. Cl.: A61B 5/00

(54) **ERFASSUNGSVORRICHTUNG FÜR VITALZEICHEN**
DETECTION DEVICE FOR VITAL SIGNS
DISPOSITIF DE DÉTECTION DE SIGNES VITAUX

(30) Priorität: 09.01.2008 AT 292008
(43) Veröffentlichungstag der Anmeldung: 27.10.2010
(73) Patentinhaber: ASMAG-Holding GmbH, 4645 Grünau im Almtal (AT)
(72) Erfinder: SCHRÖTER, Klaus, 14197 Berlin (DE)
(74) Vertreter: Ofner, Clemens
(86) Internationale Anmeldenummer: PCT/AT2009/000003
(87) Internationale Veröffentlichungsnummer: WO 2009/086575

(56) Entgegenhaltungen:
- DE-A1- 10 061 299
- DE-A1-102004 048 864
- GB-A- 2 408 209
- JP-A- 2007 313 299
- US-A- 5 228 449
- US-A1- 2003 174 049
- US-A1- 2003 181 817
- US-A1- 2008 076 974

## Beschreibung

Die Erfindung betrifft eine Erfassungsvorrichtung für Vitalzeichen.
Für eine medizinisch bedingte Dauerüberwachung einer Person, oder eine Trainingsüberwachung bzw. Protokollierung ist es erforderlich, in regelmäßigen Abständen Vitalzeichen zu erfassen. Um den Vorgang für die zu überwachende Person möglichst angenehm zu gestalten ist es von ganz besonderem Vorteil, wenn eine Überwachungsvorrichtung derart ausgebildet ist, dass sie vom Träger der Vorrichtung als nicht störend wahrgenommen wird. Insbesondere ist es von Vorteil, wenn eine derartige Überwachungsvorrichtung die Bewegungsfreiheit des Trägers nicht oder nur unwesentlich einschränkt. Ebenfalls von Vorteil ist ein möglichst langer Betrieb
In der WO 2006/005169 A1 ist eine Vorrichtung und ein Verfahren zur Überwachung von Vitalzeichen offenbart, bei der der Patient am Handgelenk eine Auswertevorrichtung trägt und wobei eine optische Erfassungsvorrichtung in der Handfläche gehalten wird. Die optische Erfassungsvorrichtung ist bspw. als Ring ausgebildet, der über einem Finger, bevorzugt dem Daumen, getragen wird.
Die WO 2005/048830 A1 offenbart eine Vorrichtung zur Erfassung von Vitalzeichen. Die Vorrichtung ist dazu in einem Gehäuse angeordnet, wobei an der Außenseite des Gehäuses metallische Kontaktelektroden angeordnet sind. Zur Erfassung von Vitalzeichen muss der Benutzer manuell die Vorrichtung an eine Stelle des Körpers anlegen. Die erfassten Daten werden bspw. über ein Weitbereichskommunikationsmittel an einen Betreuer in einem Kontrollzentrum übertragen.

Aus der JP 2007-313299A ist eine Sensorvorrichtung zur Erfassung biologischer Information bekannt, welche Sensorvorrichtung ein selbstklebendes Band aufweist und an einem Abschnitt des menschlichen Handgelenkes angebracht ist, an dem ein Puls fühlbar ist. Ferner ist eine Identifikationsmarke angeordnet, in welcher persönliche Information gespeichert ist. Vorgesehen ist ferner ein Kommunikationsschaltkreis mit einer Antenne, welche von der Klebeoberfläche abgehoben und durch eine Öffnung im Klebeband gezogen wird, wodurch ein Tausch des Erfassungssensors möglich ist, falls der Sensor schmutzig wird und ausge tauscht werden muss, ohne den Kommunikationsschaltkreis ebenfalls tauschen zu müssen. Die Antenne wird in einem Fixierungsmechanismus an der Oberfläche des selbstklebenden Bandes befestigt. Die biologische Informationserfassungsvorrichtung, eine Speichereinheit, eine arithmetische Verarbeitungseinheit sowie ein Kommunikationsschaltkreis sind jeweils basierend auf Dünnfilmtransistoren hergestellt. Falls die Antenne zu lang ist, um vom Fixierungsmechanismus aufgenommen zu werden, kann die Antenne mittels eines weiteren selbstklebenden Bandes am Handgelenk fixiert werden. Die von der Antenne verwendete Frequenz zur Übertragung der Information beträgt beispielsweise 13,56 MHz oder 2,45 GHz. Die elektrische Energie zum Betrieb der arithmetischen Verarbeitungseinheit, der Sensor-vorrichtung und der Speicher- und Kommunikationseinrichtung, wird über die Antenne bereitgestellt, wobei von der Antenne eine elektromagnetische Welle empfangen wird, die von einer externen Vorrichtung bereitgestellt wird.

Das Dokument US 2003/0174049 A1 D3 offenbart eine tragbare Identifikationsmerkmal, welche über ein drahtloses Kommunikationsnetzwerk, insbesondere über Bluetooth, mit einer Kommunikationsgegenstelle kommuniziert. Das tragbare Identifikationsmerkmal kann ferner seine Position mittels GPS erfassen und zur Gegenstelle übertragen. Das Kommunikationsnetzwerk kann ein lokales wirkendes oder weit reichendes Kommunikationsnetzwerk sein, wobei mehrere bekannte Kommunikationsmedien vorgesehen sind. Das Identifikationsmerkmal hat ein gedrucktes Leitmuster, welches das Entfernen des Identifikationsmerkmals von einem Körperteil erkennen wird, da es dadurch es zu einer Unterbrechung des leitfähigen Musters kommt. Die Antenne des Identifikationsmerkmals kann beispielsweise als Ansteckantenne oder als integrierte Antenne ausgebildet sein, wobei insbesondere Mikrostreifenleiterantennen vorgesehen sein können. Ferner kann ein globales oder lokales Positionsortungssystem vorgesehen sein. Zur Erfassung eines Patienten ist vorgesehen, dass ein Masterknoten eine Antenne oder eine Antennenanordnung aufweist, welche einen effektiven Erfassungsbereich ausbildet, sodass sich der Patient mit dem Identifikationsmerkmal im Bereich der Mastererfassungsknoten bewegen kann und ferner bei Vorhandensein mehrerer Masterknoten, von einem Masterknoten auf den nächsten Masterknoten weitergereicht wird, sobald sich das Identifikationsmerkmal im Erfassungsbereich des weiteren Masterknotens befindet. Somit ist ferner eine Ausbildung möglich, nach der der Patient innerhalb eines Bereichs von 100m rundum die Leseeinrichtung detektiert werden kann.

Dokument GB 2 408 209 A offenbart eine flexible medizinische Lichtquelle, bei der ein oder mehrere organische Lichtemittierende Dioden auf einem flexiblen Substrat angeordnet sind. Insbesondere ist offenbart, dass sogenannte Puls-Oximeter eine Lichtquelle und einen Photodetektor aufweisen, wobei durch die Ausbildung mittels organischer Leuchtdioden auf einem flexiblen Trägersubstrat, eine bessere Anpassung an den Körperteil gegeben ist, wobei die organische Leuchtdiodenanordnung eine Wellenlängen-Konvertierungsschicht aufweist, um ein blaues Emissionsspektrum in ein infrarotes Emissionsspektrum umzuwandeln.

Aus Dokument US 2003/0181817 Alist ein Sensor und eine Sensorkontrolleinrichtung zur Erfassung von Vitalzeichen bekannt, wobei der Sensor eine Energieerzeugungseinheit umfasst, welche drahtlos von einer Energieversorgungseinheit in der Empfangsvorrichtung mit Energie versorgt wird. Der Sensor überträgt sein erfasstes biometrisches Signal drahtlos an die Empfangsvorrichtung und wird von dieser mit elektrischer Energie versorgt wird. Die Energieversorgung erfolgt über eine Leistungsträgerfunkwelle, wobei die versorgende Funkwelle durch Licht gebildet wird und die Energieerzeugungseinheit einen Licht-Elektrizitäts-Konverter aufweist. Das Detektionssystem ist beispielsweise am Finger angebracht und erfasst Vitalzeichen, wie bspw. den Blutfluss, und überträgt dieses Vitalzeichen zu einer Kontrollvorrichtung, die beispielsweise am Armgelenk angebracht ist und eine elektromagnetische Energieübertragungswelle aussendet, um die Sensorvorrichtung mit Energie zu versorgen. Insbesondere ist eine Antennenspule vorhanden, die elektrische Energie von außerhalb des Sensors empfängt und Vitalzeichen aussendet. Die Antennenspule der Energieerzeugungsvorrichtung basiert auf elektromagnetischer Induktion bzw. elektromagnetischen Kopplungseffekten.

Dokument DE 100 61 299 A1 offenbart eine Vorrichtung zur Erfassung und/oder Weiterleitung zumindest eines Umwelteinflusses, welche Vorrichtung im Wesentlichen aus organischem Funktions-material aufgebaut ist. Die Vorrichtung kann beispielsweise als RFID-Marke ausgebildet sein, wobei im Sensor ein Umwelteinfluss, eine Änderung eines Widerstandes und/oder eine Erzeugung einer Spannung initiiert, welche Änderung von organischen Feldeffekttransistoren entweder digital oder analog zum Signalausgang weitergeleitet wird.

Aus Dokument DE 10 2004 048 864 A1 ist ein analytisches Testsystem mit drahtloser Datenübertragung zur Bestimmung der Konzentration eines Analyts in einer Körperflüssigkeit be kannt. Dieses Testelement weist einen Transponder zur drahtlosen Übertragung von chargenspezifischen Daten und/oder Messwerten auf. Um zu gewährleisten, dass bei jeder Messung vertauschungssicher die korrekten Kalibrationsdaten verwendet werden, sind bei jedem Teststreifen die Chargenkennung und die Kalibrationsdaten gespeichert und lassen sich über den Transponder auslesen. Das Aufbringen eines Transponders auf das analytische Testelement erfolgt in einem, einfach in einen Prozessschritt zu integrierenden Rolle-zu-Rolle-Produktionsschritt. Zusätzlich kann vorgesehen sein, die analytischen Testelemente auch noch mit einer eindeutigen Identifikationsnummer zu versehen, um so jeden einzelnen Teststreifen beschreiben und abfragen zu können. Es ist offenbart, dass ein im Körper implantiertes Testelement mit einem extrakorporalen Lesemodul kommuniziert, wobei hier die Reichweite ca. 10-20cm beträgt. Ferner ist offenbart, dass beispielsweise mit externen Einheiten über eine Luft-Kommunikationsstrecke bis zu einem Meter kommuniziert werden kann, hier in einem Frequenzbereich von 13,56 MHz. Für größere Kommunikationsentfernungen ist offenbart, dass Frequenzen im Bereich von 800 bis 2000 MHz verwendet werden, wodurch eine Entfernung von bis zu 4m überbrückt werden kann. Bei einem Transpondersystem werden die zu übertragenden Daten auf jener Energieversorgungsquelle aufmoduliert, die zur Versorgung des Transpondersystems erforderlich ist.
Die aus dem Stand der Technik bekannten Vorrichtungen haben den Nachteil, dass bspw. eine Vorrichtung am Handgelenk getragen wird und das Erfassungsmittel in der Hand gehalten werden muss. Ebenso von Nachteil ist, wenn die Vorrichtung zur Durchführung der Messung vom Benutzer am Körper angeordnet werden muss, bspw. durch andrücken. Ebenso haben die bekannten Vorrichtungen zumeist eine nicht unbeträchtliche Größe und schränken somit die Bewegungsfreiheit des Trägers deutlich ein, oder erlauben während der Messung nur einen stark eingeschränkten Handlungsspielraum.

Die Aufgabe der Erfindung liegt nun darin eine autarke Vorrichtung zu schaffen, mit der sich über einen langen Zeitraum, kontinuierlich Vitalzeichen erfassen lassen, wobei ein Mittel zur Erfassung der Vitalzeichen derart am Körper des Trägers positioniert werden kann, dass es zu keiner bzw. nur zu einer sehr geringen Beeinträchtigung der Bewegungsfreiheit des Trägers kommt.
Die Aufgabe der Erfindung wird durch die Erfassungsvorrichtung gemäß Anspruch 1 gelöst, bei der der Sensor als elastisch rückstellbar verformbarer Dünnfilmsensor ausgebildet ist und die über eine Kommunikationsverbindung zur Übertragung von Energie und Messwerten bzw. Messdaten verfügt. Bei einem Dünnfilmsensor werden die einzelnen aktiven Schichten mittels Aufdampfen und/ oder Aufdrucken auf ein Substrat aufgebracht. Der wesentliche Vorteil liegt darin, dass sich diese dünnen Schichten besonders gut an die Verformungen des Sensors anpassen können. Da der Sensor der erfindungsgemäßen Erfassungsvorrichtung an Körperteilen angeordnet ist und durch die Bewegung des Körper auch dynamischen Verformungen ausgesetzt ist, ist es für einen zuverlässigen Betrieb des Sensors von ganz entscheidender Bedeutung, wenn der Sensor durch die mechanische Beanspruchung während des bestimmungsgemäßen Einsatzes nicht beschädigt wird.
Ebenfalls im Hinblick auf den Tragekomfort und damit die Akzeptanz durch den Benutzer ist es, wenn über die Kommunikationsverbindung elektrische Energie und Daten übertragen werden können. Die Erfassungsmittel benötigen zur Erfassung der Vitalzeichen elektrische Energie, die am Sensor bereitgestellt werden muss, wobei dies üblicherweise mittels einer Energiequelle geschieht. Bekannte Energiequellen haben jedoch den Nachteil dass sie kapazitätsbedingt, ein nicht unbeträchtliches Volumen und Gewicht aufweisen und somit bei einer Anordnung am Sensor den Tragekomfort einschränken und daher der Benutzer den Sensor als störendes Teil empfindet. Eine kleinvolumige Energiequelle hat aufgrund der geringen Kapazität den Nachteil einer sehr begrenzten Einsatzdauer. Da die elektrische Energie über die Kommunikationsverbindung an den Sensor übertragen wird, lässt sich der Sensor wesentlich kompakter und leichter ausbilden, ohne die Einsatzdauer der erfindungsgemäßen Erfassungsvorrichtung wesentlich zu beschränken.
Einen ganz besonderen Vorteil erhält man, wenn die erste Kommunikationsverbindung durch das Nahfeld der Haut gebildet ist. Diese Ausbildung ermöglicht es, den Sensor an einer beliebigen Stelle des Körpers anzuordnen, ohne sich über eine Verdrahtung kümmern zu müssen. Insbesondere hat die Ausbildung den Vorteil, dass der Sensor bevorzugt an jenen Körperpositionen angeordnet werden kann, wo die zu erfassenden Vitalzeichen besonders ausgeprägt erfassbar sind. Ebenso kann der Sensor besonders geschützt angeordnet sein bzw. ist die Anordnung derart möglich, dass die Bewegungsfreiheit des Trägers nicht eingeschränkt wird. Dies ist insbesondere bei einer Erfassung von Trainingsdaten von Vorteil, da bei entsprechender Anordnung des Sensors, der natürliche Bewegungsablauf des Trainierenden nicht eingeschränkt wird.

Weiters von Vorteil ist, dass sich die Reichweite der ersten Kommunikationsverbindung derart einstellen lässt, dass eine Störung durch eine zweite Erfassungsvorrichtung die von einem weiteren Patienten getragen wird verhindert wird.

In einer vorteilhaften Weiterbildung kann die erste Kommunikationsverbindung auch durch eine Hochfrequenzverbindung geringer Reichweite gebildet sein, bspw. durch eine HF-Verbindung im lizenzfreien ISM-Bereich (Industrial, Scientific, and Medical Band) von 868 bzw. 915MHz. Für eine Kommunikationsverbindung in diesem Frequenzbereich ist aufgrund der großen Verbreitung bereits eine Vielzahl von Kommunikationsvorrichtungen wie Sender/ Empfängerschaltungen verfügbar.

Eine vorteilhafte Weiterbildung erlaubt die Bestimmung des Sauerstoffgehalts des Blutes mittels Pulsoximetrie. Zur Durchführung des Pulsoximetrieverfahrens ist ein Erfassungsmittel erforderlich, das anspruchsgemäß durch eine Quelle für elektromagnetische Strahlung und einen elektromagnetischer Strahlungsdetektor gebildet ist. Bei diesem Verfahren werden Kapillargefäße mit Licht unterschiedlicher Wellenlänge beleuchtet und die jeweils unterschiedlichen Absorptionsraten miteinander in Beziehung gesetzt. Durch Verhältnisbildung und Vergleich mit Referenzwerten lässt sich dann der Sauerstoffsättigungsgrad ermitteln, wobei diese Auswertung bspw. in einer externen Auswertevorrichtung durchgeführt wird. Zur Durchführung der Messung wird bspw. Licht mit einer Wellenlänge im Bereich von 660nm und im Bereich von 940nm eingesetzt. Gegebenenfalls kann auch Umgebungslicht eingesetzt werden.

Als Strahlungsquelle können bspw. alle Licht aussendenden Halbleiterbauteile verwendet werden, bevorzugt werden Leuchtdioden eingesetzt. Als Detektor wird bevorzugt ein Fototransistor eingesetzt, jedoch sind auch alle anderen fotoempfindlichen elektronischen Bauteile einsetzbar.

In Weiterbildungen kann das Erfassungsmittel auch Vitalzeichen erfassen wie bspw. den Blutdruck oder den Blutzuckergehalt. Wesentlicher Vorteil dieser Weiterbildungen ist, dass auch diese Vitalzeichen nichtinvasiv erfasst werden, insbesondere erlaubt ein anspruchsgemäß ausgebildetes optisch arbeitendes Detektionssystem eine Erfassung derartiger Vitalzeichen. Auch ist ein Erfassungsmittel denkbar, um die Atemfrequenz erfassen zu können.

Einen ganz besonderen Vorteil erhält man, wenn das Erfassungsmittel durch organische und/ oder anorganische Halbleiterbauteile gebildet ist. Im Hinblick auf den bevorzugten Einsatzzweck des Sensors haben Erfassungsmittel, die durch organische Halbleiterbauteile gebildet sind den besonderen Vorteil, dass sie besonders einfach, schnell und kostengünstig hergestellt werden können. Hinsichtlich der Umweltproblematik bei der Herstellung und bei der Entsorgung haben organische Halbleiter den weiteren Vorteil, dass bei der Herstellung keine energieaufwändigen Prozesse bspw. Hochtemperaturprozesse, erforderlich sind und eine Entsorgung des Sensors ohne Gefahr für die Umwelt möglich ist. Bei einer kontinuierlichen Erfassung von Vitalsignalen kann es zu einer Verschmutzung der Erfassungsmittel des Sensors kommen, wodurch die Funktionalität des Sensors eingeschränkt wird, bzw. dieser unbrauchbar wird. Gemäß den genannten Vorteilen lässt sich nun ein so genannter Einwegsensor ausbilden, wodurch eine Reinigung eines verschmutzten Sensors nicht mehr erforderlich ist und dieser sofort gegen einen neuen Sensor ausgetauscht wird.

Organische Halbleiterbauteile und insbesondere organische Leuchtdioden und organische Fototransistoren sind aus dem Stand der Technik hinlänglich bekannt, sodass hier auf eine weitere Beschreibung verzichtet wird.

In einer vorteilhaften Weiterbildung kann das Erfassungsmittel aber auch durch anorganische Halbleiterbauteile, sowie eine Kombination aus organischen und anorganischen Halbleiterbauteilen gebildet sein.

Gemäß einer Weiterbildung ist der Sensor durch eine flächenhafte Trägerlage gebildet, auf der das Erfassungsmittel angeordnet ist. Eine elastisch rückstellbar verformbar ausgebildete flächenhafte Trägerlage eines Sensors hat den Vorteil, dass sie sich besonders gut an die Oberflächenstruktur anpassen kann, auf der der Sensor angeordnet wird. Die Trägerlage kann bspw. aus PEN oder PET gebildet sein.

In einer vorteilhaften Weiterbildung ist die flächenhafte Trägerlage elektrisch isolierend ausgebildet, wodurch sich das Erfassungsmittel ohne zusätzliche elektrisch isolierende Schutzschicht auf die Trägerlage aufbringen lässt. Weiters von Vorteil ist, dass die Trägerlage bevorzugt als Folienmaterial auf einer Rolle vorliegt, wodurch sich besonders rationelle Verfahren zur Herstellung des Sensors einsetzen lassen, insbesondere Durchlaufverfahren.

Gemäß einer vorteilhaften Weiterbildung kann das optisch wirkende Erfassungsmittel auch auf der, dem Körperteil abgewandten Flachseite der Trägerlage angeordnet werden. Wenn die Trägerlage zumindest in dem Spektralbereich der Strahlungsquelle bzw. des Strahlungsdetektors transparent bzw. semitransparent ist, ist es nicht erforderlich, die Erfassungsmittel der Körperoberfläche zugeordnet anzuordnen, wo sie einer erhöhten Schmutzbelastung ausgesetzt sind.

Einen ganz entscheidenden Vorteil erhält man, wenn das Erfassungsmittel auf einer Flachseite der Trägerlage aufgedruckt. Durch Druckverfahren hergestellte Erfassungsmittel, bspw. durch Tintenstrahldruck, Siebdruck, Stempeldruck sind besonders kostengünstig verfügbar. Druckverfahren haben den ganz besonderen Vorteil, dass sie sich sehr schnell und flexibel an geänderten Anforderungen anpassen lassen, wodurch auch geringe Stückzahlen wirtschaftlich herstellbar sind. Auch lassen sich komplexe Strukturen wie sie für Halbleiterbauteile erforderlich sind mit Druckverfahren bedeutend einfacher und schneller realisieren. Insbesondere lässt sich das Erfassungsmittel auf nachträglich auf vorgefertigte Komponenten aufdrucken.

Gemäß einer vorteilhaften Weiterbildung ist das Erfassungsmittel durch organische Halbleiterbauteile gebildet. Organische Halbleitermaterialien lassen sich besonders vorteilhaft in Druckverfahren einsetzen, insbesondere sind Anordnungen ausbildbar, die mit anorganischen Halbleitermaterialien nicht oder nur sehr schwierig möglich wären. Beispielsweise kann bei einem ersten Druckvorgang ein Abschnitt freigelassen werden, in dem in einem zweiten Druckvorgang ein anderes Material gedruckt wird. Bei anorganischen Materialien wären dazu aufwändig Lithografie und Ätzprozesse erforderlich.
Im Hinblick auf eine Benutzerfreundlichkeit ist eine Ausbildung von Vorteil, bei der Sensor einfach angelegt bzw. abgenommen werden kann. Wie allgemein bekannt, ist eine Manschette derart ausgebildet, dass sie in ihrer Längserstreckung zumindest die Länge aufweist, die zur Umfassung des geforderten Umfangs erforderlich ist. Die Manschette kann als geschlossene zylinderartige Manschette ausgebildet sein, wobei die Feinanpassung an den Umfang bevorzugt durch elastische Abschnitte geschieht. Die Manschette kann aber auch als abgewickelter Zylinder ausgebildet sein, in diesem Fall wird die Manschette auf den zu umfassenden Umfang aufgelegt und mittels eines Haltemittels auf diesem positioniert. Die erfindungsgemäße Erfassungsvorrichtung verfügt über eine Manschette, die ein Verschlusselement aufweist, das zur Abgabe eines Triggersignals ausgebildet ist. Die Durchführung der Messung der Vitalsignale ist nur dann erforderlich, wenn der Sensor auf einem Körperteil angebracht ist. Das Triggersignals wird dazu verwendet, die Erfassungsvorrichtung zu aktivieren, wodurch hernach periodisch Vitalsignale erfasst werden. Diese Ausbildung verhindert in vorteilhafter Weise Fehlmessungen. Beispielsweise kann sich durch eine Unachtsamkeit der Sensor vom Körper lösen, woraufhin in weiterer Folge keine Vitalsignale mehr erfasst werden und ein derartiger Zustand bspw. einen Alarm auslösen könnte. Anspruchsgemäß würde jedoch das unbeabsichtigte Lösen des Sensors vom Körper ein Triggersignal erzeugen und somit würde eine Alarmierung aufgrund fehlender Vitalzeichen unterdrückt. Das durch ein unbeabsichtigtes Lösen des Sensors ausgelöste Triggersignal kann jedoch auch dazu verwendet werden, den Träger über diesen Umstand zu informieren. Die anspruchsgemäße Ausbildung bringt somit eine bedeutende Erhöhung der Betriebssicherheit und des Komforts für den Benutzer.
Erfindungsgemäß erfolgt die Übertragung von Energie von dem Energieversorgungsmodul zum Sensor über eine drahtlose Kommunikationsverbindung. Ist nun im ersten Kommunikationsmodul ein Speicher für elektrische Energie vorhanden, sind auch solche Erfassungsmittel am Sensor anordenbar, die einen höheren Energieverbrauch aufweisen. Erfindungsgemäß werden Vitalzeichen nicht kontinuierlich erfasst, sondern nur in einem vorwählbaren Zeitraster, bspw. jede Minute. Da über die drahtlose Kommunikationsverbindung nur eine begrenzte Menge Energie übertragen werden kann, hat die anspruchsgemäße Ausbildung den Vorteil, dass der Energiespeicher in den Pausen zwischen den Messungen aufgeladen werden kann. Während der Messung werden die Erfassungsmittel vom Energiespeicher mit elektrischer Energie versorgt. Die Messzyklen sind dabei so zu wählen, dass die Zeit zwischen den Messvorgängen ausreicht, den Energiespeicher mit ausreichend Energie zu laden, um den nächsten Messvorgang durchführen zu können.

Der Energiespeicher ist bspw. als kapazitiver Speicher oder als elektrochemisches Speicherelement ausgebildet. Besonders vorteilhaft ist eine Ausbildung bei der der Energiespeicher als Dünnfilmelement ausgebildet ist bspw. als Polymerakku, da sich derartige Energiespeicher mittels Druckverfahren herstellen lassen.

Gemäß einer vorteilhaften Weiterbildung weist das erste und zweite Kommunikationsmodul eine Sende- und/oder Empfangsvorrichtung für elektromagnetische Strahlung auf. Die Sende- und/oder Empfangsvorrichtung ist jeweils dazu ausgebildet, elektrische Energie, Steuersignale sowie ermittelte Messwerte die am ersten bzw. zweiten Kommunikationsmodul anliegen, derart in ein elektromagnetisches Feld überzuführen, dass die drahtlose Kommunikationsverbindung zwischen den ersten und zweiten Kommunikationsmodul ausgebildet wird.

Gemäß einer beanspruchten Weiterbildung ist die Kommunikationsverbindung durch das Nahfeld der Haut gebildet. Die Sende- und/oder Empfangsvorrichtung des ersten und zweiten Kommunikationsmoduls muss in diesem Fall derart ausgebildet sein, dass eine Ankopplung der elektromagnetischen Strahlung an das Transportmedium Haut ermöglicht wird. Da neben Messwerten bzw. Steuerungssignalen auch noch elektrische Energie zur Versorgung des Sensors über die Kommunikationsverbindung übertragen werden soll, hat sich eine Frequenz von 100kHz für das elektromagnetische Wechselsignal als besonders vorteilhaft erwiesen.

Die Sende- und/oder Empfangsvorrichtung kann bspw. durch eine Antenne gebildet sein bspw. durch eine gedruckte Streifenleiterantenne. Eine derart ausgebildete Sende- und/oder Empfangsvorrichtung lässt sich besonders vorteilhaft am Sensor anordnen bzw. in diesem integrieren.

Von Vorteil ist eine Ausbildung, bei der die Energiequelle durch ein Element aus der Gruppe umfassend chemisches Element, anorganische bzw. organische Solarzelle gebildet ist. Da das Energieversorgungsmodul vom Sensor distanziert angeordnet ist, also die Bewegungsfreiheit des Trägers kaum einschränkt, kann als Energiequelle auch eine kapazitätsstärkere Vorrichtung eingesetzt werden. Insbesondere lassen sich weit verbreitete, kostengünstige und sehr kapazitätsstarke chemische Elemente einsetzen. Für einen energieautarken Betrieb der erfindungsgemäßen Erfassungsvorrichtung ist es aber auch von Vorteil, wenn die Energiequelle als Solarzelle, insbesondere als organische Solarzelle, ausgebildet ist. Organische Solarzellen haben den besonderen Vorteil, dass sie besonders kostengünstig und rationell herstellbar sind, insbesondere lassen sie sich mittels Druckverfahren herstellen.

Von bedeutendem Vorteil der anspruchsgemäßen Ausbildung ist, dass die Versorgung des Sensors mit elektrischer Energie durch das Energieversorgungsmodul erfolgt. Da am Sensor keine Energiequelle vorhanden sein muss, kann dieser besonders kompakt ausgebildet sein, was für eine hohe Akzeptanz des Trägers von Vorteil ist, da ein kompakter Sensor die Bewegungsfreiheiten nicht oder nur kaum einschränkt und daher nicht als störend empfunden wird.

Zur Aufbereitung der erfassten Messwerte ist es von Vorteil, wenn das Energieversorgungsmodul eine Auswertevorrichtung aufweist. Die vom Erfassungsmittel erfassten Vitalsignale liegen als so genannte Rohdaten vor, die vor einer datentechnischen Erfassung aufbereitet werden müssen. Diese Aufbereitung kann nun in besonders vorteilhafter Weise bereits von der Auswertevorrichtung des Energieversorgungsmoduls übernommen werden. Bei der Erfassung des Sauerstoffgehalts des Bluts werden bspw. Helligkeitswerte erfasst. Nach dem bekannten Verfahren der Pulsoximetrie müssen die einzelnen Helligkeitswerte bei unterschiedlichen Spektralkomponenten zueinander in Verhältnis gesetzt werden, was von der Auswertevorrichtung übernommen werden kann, wodurch bspw. die Auswertevorrichtung einen, dem Sauerstoffgehalt proportionalen Werte zur Verfügung stellt. Die Auswertevorrichtung kann aber auch bspw. dazu ausgebildet sein, die periodisch erfassten Vitalzeichen zu agreggieren, um so einen Langzeittrend ermitteln zu können. In einer vorteilhaften Weiterbildung kann die Auswertevorrichtung bspw. auch eine Alarmvorrichtung aufweisen, die den Träger bei einer Über- bzw. Unterschreitung eines Grenzwertes eines Vitalzeichens alarmiert.

Zur Durchführung der Messwertaufbereitung ist es von Vorteil, wenn die Auswertevorrichtung eine Steuerungseinrichtung und einen Speicher umfasst. Im Speicher können Vorschriften hinterlegt sein, die von der Steuerungseinrichtung geladen und ausgeführt werden, um so bspw. die Aufbereitung der erfassten Vitalzeichen durchzuführen. Die Steuerungseinrichtung kann bspw. durch einen Mikrocomputer gebildet sein, der Speicher kann durch einen flüchtigen bzw. nicht flüchtigen Speicherbaustein gebildet sein. Auch kann de Speicher dazu ausgebildet sein, erfasste und aufbereitete Vitalzeichen bis zur nächsten Datensynchronisation zwischenzuspeichern.

Die erfindungsgemäße Erfassungsvorrichtung kann eigenständig betrieben werden, die Überwachung, Auswertung und ggf. Alarmierung des Trägers geschieht dann autark, ohne einen Eingriff bzw. eine Beteiligung einer datentechnischen Auswertevorrichtung. Die erfindungsgemäße Erfassungsvorrichtung kann aber auch dazu ausgebildet sein, die personenspezifischen Vitaldaten von einer Analysevorrichtung auswerten zu lassen. Daher ist es von besonderem Vorteil, wenn das Energieversorgungsmodul ein drittes Kommunikationsmodul aufweist. Dieses dritte Kommunikationsmodul ist bevorzugt zur Herstellung einer drahtlosen Kommunikationsverbindung ausgebildet, jedoch ist auch eine kabelgebundene Anbindung an eine Analyseeinrichtung denkbar. Das dritte Kommunikationsmodul ist jedenfalls dazu ausgebildet, die erfassten und ggf. aufbereiteten Messwerte der Vitalsignale in eine übertragbare Form umzuwandeln.

Gemäß einer vorteilhaften Weiterbildung ist das dritte Kommunikationsmodul zur Herstellung einer Datenverbindung mit einer Datengegenstelle einer Datenanalyseeinrichtung ausgebildet. Die Datenverbindung kann dabei durch eine, im Nahbereich wirkende, drahtlose Kommunikationsverbindung gebildet sein, bspw. RFID, Bluetooth, IrDA. Das dritte Kommunikationsmodul sowie die Datengegenstelle weisen dabei eine Sende- und/oder Empfangsvorrichtung auf. Die Datenverbindung kann jedoch auch durch eine kabelgebundene Kommunikationsverbindung gebildet sein, bspw. USB.

Eine vorteilhafte Weiterbildung erhält man, wenn anspruchsgemäß das dritte Kommunikationsmodul ein Speichermodul aufweist. Zur Übertragung der erfassten Vitalsignale an die Datenanalyseeinrichtung muss sich der Benutzer, insbesondere jedoch das Energieversorgungsmodul, in der Nähe der Datengegenstelle der Datenanalyseeinrichtung befinden, um die Datenverbindung herzustellen. Wenn das dritte Kommunikationsmodul ein Speichermodul aufweist, können die erfassten Vitalsignale eine, durch die Kapazität des Speichermoduls festlegbare Zeit zwischengespeichert werden und so erst später, bei einer hergestellten Datenverbindung an die Datenanalyseeinrichtung übermittelt werden. Dies ist insbesondere dann von Vorteil, wenn über möglichst lange Zeiträume Vitalzeichen erfasst werden sollen, da es dem Träger der erfindungsgemäßen Erfassungsvorrichtung nicht zugemutet werden kann, übermäßig oft die Datenverbindung mit der Datenanalyseeinrichtung herzustellen um die erfassten Vitalzeichen zu synchronisieren. Besonders bevorzugt ist eine Ausbildung, bei der das Speichermodul den gespeicherten Inhalt auch ohne Versorgung mit elektrischer Energie behält.

Eine Anordnung des Energieversorgungsmoduls in bzw. an der Kleidung des Benutzers hat den Vorteil, dass eine möglichst unauffällige, die Bewegungsfreiheit des Benutzers möglichst nicht einschränkende Anordnung des Energieversorgungsmoduls möglich ist. Gegebenenfalls kann das Energieversorgungsmodul auch auf der Körperoberfläche angeordnet sein, bspw. durch Fixieren mit einem elastischen Halteband. Eine Anordnung des Energieversorgungsmoduls auf der Körperoberfläche hat insbesondere dann den Vorteil, wenn die Kommunikationsverbindung durch das Nahfeld der Haut gebildet ist, da dann eine besonders gute An- bzw. Auskopplung der elektromagnetischen Felder an die Hautoberfläche möglich ist. Für eine Kommunikationsverbindung durch das Nahfeld der Haut ist jedoch ein direkter Hautkontakt nicht erforderlich, insbesondere ist es ausreichend, wenn das Energieversorgungsmodul, insbesondere das zweite Kommunikationsmodul, in einem Nahbereich von mehreren cm Abstand von der Hautoberfläche angeordnet ist.

Im Hinblick auf einen möglichst langen, autarken Betrieb der Erfassungsvorrichtung und insbesondere im Hinblick auf einen möglichst kompakten Sensor, ist es von ganz besonderem Vorteil, wenn das Energieversorgungsmodul zur Aktivierung des Sensors ausgebildet ist. Vitalzeichen müssen nicht kontinuierlich erfasst werden, insbesondere reicht eine periodische Erfassung, bspw. jede Minute. Um den Sensor kompakt und möglichst energiesparend ausbilden zu können, ist es daher von Vorteil, wenn diese Zeitsteuerung nicht am Sensor implementiert ist und daher permanent Energie verbrauchen würde, sondern der Sensor von einem externen Zeitgeber aktiviert wird. Das Energieversorgungsmodul kann daher bspw. einen Zeitgeber aufweisen, der periodisch über die Kommunikationsverbindung den Sensor aktiviert. Der Zeitgeber ist bevorzugt in der Auswertevorrichtung angeordnet sein bzw. von dieser gebildet sein. Die Aktivierung kann bspw. dadurch erfolgen, dass vom zweiten Kommunikationsmodul ein Signal mit einer speziellen Frequenz ausgesandt wird, auf die eine Aktivierungsvorrichtung im ersten Kommunikationsmodul selektiv ist und hernach das bzw. die Erfassungsmittel aktiviert. Nach der Erfassung der Vitalzeichen werden diese über die Kommunikationsverbindung an das Energieversorgungsmodul übertragen und der Sensor wird wieder deaktiv. Die Energie verbrauchende Erfassung der Vitalzeichen ist daher nur einen kurzen Zeitraum der gesamten Betriebszeit aktiv, wodurch man, aufgrund des geringeren Energieverbrauchs, eine bedeutende Verlängerung der Einsatzdauer erreicht und somit auch kompaktere Bauweisen möglich sind.

Die Erfindung wird im nachfolgenden anhand der in den Zeichnungen dargestellten Ausführungsbeispiele näher erläutert.

Es zeigen jeweils in schematisch vereinfachter Darstellung:
- Fig. 1: die erfindungsgemäße Erfassungsvorrichtung;
- Fig. 2: ein Prinzipbild der Synchronisation der erfassten Vitalzeichen mit einer Datenanalyseeinrichtung.

Einführend sei festgehalten, dass in den unterschiedlich beschriebenen Ausführungsformen gleiche Teile mit gleichen Bezugszeichen bzw. gleichen Bauteilbezeichnungen versehen werden, wobei die in der gesamten Beschreibung enthaltenen Offenbarungen sinngemäß auf gleiche Teile mit gleichen Bezugszeichen bzw. gleichen Bauteilbezeichnungen übertragen werden können. Auch sind die in der Beschreibung gewählten Lageangaben, wie z.B. oben, unten, seitlich usw. auf die unmittelbar beschriebene sowie dargestellte Figur bezogen und sind diese bei einer Lageänderung sinngemäß auf die neue Lage zu übertragen. Weiters können auch Einzelmerkmale oder Merkmalskombinationen aus den gezeigten und beschriebenen, unterschiedlichen Ausführungsbeispielen für sich eigenständige, erfinderische oder erfindungsgemäße Lösungen darstellen.

Sämtliche Angaben zu Wertebereichen in gegenständlicher Beschreibung sind so zu verstehen, dass diese beliebige und alle Teilbereiche daraus mit umfassen, z.B. ist die Angabe 1 bis 10 so zu verstehen, dass sämtliche Teilbereiche, ausgehend von der unteren Grenze 1 und der oberen Grenze 10 mitumfasst sind, d.h. sämtliche Teilbereich beginnen mit einer unteren Grenze von 1 oder größer und enden bei einer oberen Grenze von 10 oder weniger, z.B. 1 bis 1,7, oder 3,2 bis 8,1 oder 5,5 bis 10.

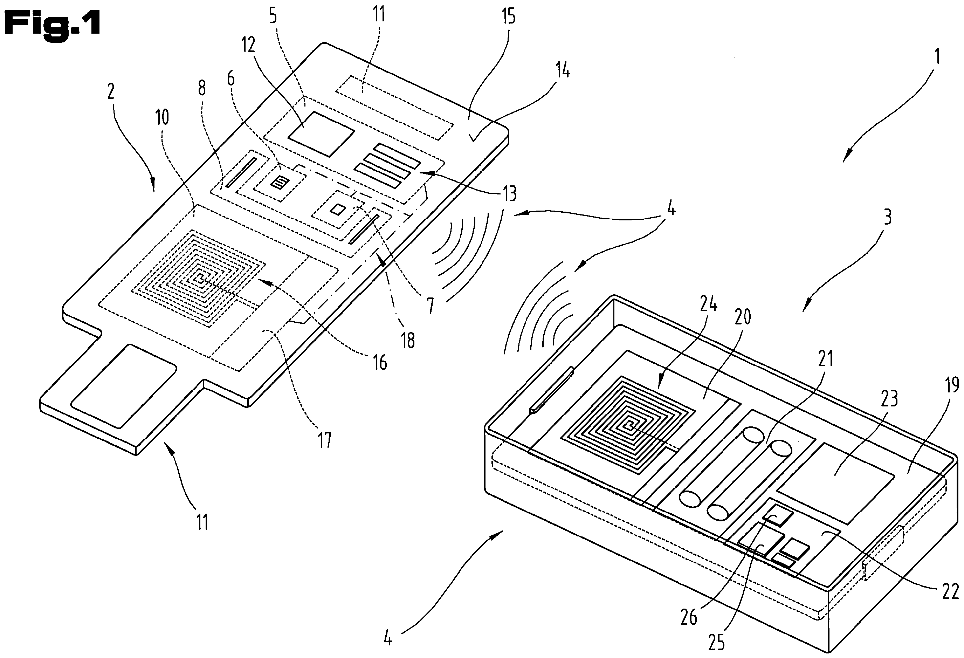

Fig. 1 zeigt die erfindungsgemäße Erfassungsvorrichtung 1, umfassend einen Sensor 2 und ein Energieversorgungsmodul 3, wobei zwischen diesen beiden eine drahtlose kurzreichweitige Kommunikationsverbindung 4 besteht.

Der Sensor 2 weist bspw. ein Erfassungsmittel für den Sauerstoffgehalt des Bluts 5, ein Erfassungsmittel für die Körpertemperatur 6, ein Erfassungsmittel für die Hautfeuchte 7 und ein Erfassungsmittel für die Leitfähigkeit der Haut 8 auf. Der Sensor 2 umfasst weiters ein erstes Kommunikationsmodul 10 und ggf. ein Verschlusselement 11.

Das Erfassungsmittel für den Sauerstoffgehalt des Bluts 5 ist durch einen elektromagnetischen Strahlungsdetektor 12 und zumindest eine Quelle für elektromagnetische Strahlung 13 gebildet. Das Erfassungsmittel 5 ermittelt den Sauerstoffgehalt des Bluts mittels des Verfahrens der Pulsoximetrie. Dabei wird von einer Strahlungsquelle 13 eine elektromagnetische Strahlung mit einer ersten Wellenlänge im optisch sichtbaren Bereich, bspw. im Bereich von 660nm ausgesandt, von einer zweiten Strahlungsquelle wird eine elektromagnetische Strahlung in einem anderen Wellenbereich, bspw. im Bereich von 940nm ausgesandt. Durch das mit Sauerstoff gesättigte Hämoglobin wird die Strahlung jeweils unterschiedlich stark abgeschwächt. Der Strahlungsdetektor 12 ist im gesamten Wellenbereich des von der Strahlungsquelle 13 ausgesandten Lichts fotosensitiv und erzeugt entsprechend der Intensität der einfallenden elektromagnetischen Strahlung, ein proportionales Ausgangssignal bspw. eine elektrische Spannung. Der Detektor kann aber auch seinen Widerstand oder eine andere elektrische Kenngröße in Abhängigkeit der Intensität der einfallenden elektromagnetischen Strahlung ändern. Die Strahlungsquelle 13 ist bevorzugt als Leuchtdiode (LED) gebildet, der Strahlungsdetektor 12 ist bevorzugt als Quantendetektor ausgebildet, bspw. als Fotodetektor oder Fotodiode. Gemäß einer besonders bevorzugten Ausbildung ist die Strahlungsquelle 13 und der Strahlungsdetektor 12 durch organische Halbleiter bzw. organische Halbleiterbauteile gebildet, die auf eine Flachseite 14 der Trägerlage 15 aufgedruckt werden. Als Druckverfahren zur Herstellung organischer Halbleiter sind bspw. Tintenstrahldruck, Siebdruck, Stempeldruck bekannt. Die Vorteile eines Druckverfahrens liegen darin, dass die auszubildenden Bauteile bzw. Strukturen besonders einfach, kostengünstig und individuell ausbildbar herstellbar sind. Druckverfahren erfordern insbesondere keine energieintensiven Herstellungsschritte, bspw. eine Bedampfung im Hochvakuum, auch lassen sich mittels eines Druckverfahrens Schichtaufbauten aus Halbleitermaterialien herstellen, die für anorganische Halbleiter nicht oder nur sehr aufwendig zu realisieren sind. Insbesondere können die auszubildenden Bauteile auch nachträglich auf Halbfertigteile aufgedruckt werden.

Das Erfassungsmittel für die Körpertemperatur ist bspw. durch ein elektronisches Bauteil gebildet, bei dem der Wert zumindest einer elektrischen Kenngröße temperaturabhängig ist. Beispielsweise lassen sich Heiß- oder Kaltleiter einsetzen, es sind aber auch elektronische Halbleiterbauteile zur Temperaturmessung geeignet, da die Strom/Spannungskennlinie eines Halbleiterübergangs temperaturabhängig ist.

Das Erfassungsmittel für die Hautfeuchtigkeit kann bspw. dadurch gebildet sein, dass eine Infrarot-Strahlungsquelle, bspw. eine IR-LED, Licht aussendet, das auf die Haut auftrifft und von den oberen Hautschichten reflektiert wird. Je höher der Wassergehalt der Hautschicht ist, desto mehr Infrarot-Licht wird reflektiert. Durch eine entsprechende Ausbildung bzw. Anordnung der Strahlungsquelle 13 und des Detektors 12 des Erfassungsmittels für den Sauerstoffgehalt des Bluts 5, kann die Erfassung der Hautfeuchtigkeit auch vom Erfassungsmittel 5 durchgeführt werden.

Zur Bestimmung der Leitfähigkeit der Haut wird bspw. an zwei Elektroden des Erfassungsmittels 8 eine ungefährliche elektrische Spannung angelegt und der fließende elektrische Strom durch die Haut gemessen. In einer vorteilhaften Weiterbildung lässt sich die Leitfähigkeit der Haut auch über die Bestimmung des Wassergehalts der Haut durch das Erfassungsmittel 7 bestimmen.

Am Sensor 2 ist weiters das erste Kommunikationsmodul 10 angeordnet. Die Sende- und/oder Empfangsvorrichtung 16 ist bevorzugt als Antenne zur Abstrahlung bzw. zum Empfang von elektromagnetischer Strahlung im freien ISM-Bereich von 868 bzw. 915MHz ausgebildet. Ein ggf. im ersten Kommunikationsmodul angeordneter Energiespeicher ist bspw. als Doppelschichtkondensator ausgebildet. Dieser weist eine sehr hohe Energiedichte und eine sehr geringe Selbstentladung auf. Das Kommunikationsmodul 10 bzw. der Energiespeicher 17 ist zur Versorgung der Erfassungsmittel 5, 6, 7, 8 mit elektrischer Energie ausgebildet. Die einzelnen Erfassungsmittel sind dabei über eine elektrische Verbindungsleitung 18 mit dem Kommunikationsmodul 10 bzw. den Energiespeicher 17 verbunden.

Der Sensor 2 ist als Manschette ausgebildet die um einen Körperteil angelegt wird, wobei zur Fixierung der Manschette auf dem Körperteil die Manschette mittels eines Verschlusselements 11 verschlossen wird. Beim Verschließen der Manschette wird vom Verschlusselement 11 ein Triggersignal erzeugt bzw. abgegeben, dass über die Kommunikationsverbindung 4 an das Energieversorgungsmodul 3 übermittelt wird. Durch dieses Triggersignal erkennt das Energieversorgungsmodul das der Sensor angelegt wurde, insbesondere wird erkannt, wenn der Sensor abgenommen wurde. Diese Ausbildung hat den Vorteil, dass die Erfassung von Vitalzeichen nur dann durchgeführt wird, wenn der Sensor angelegt ist. Insbesondere wird somit auch eine irrtümliche Fehlalarmierung bei Fehlen bzw. Ausfall der Vitalzeichen vermieden, wenn die Manschette abgenommen wurde.
Die Trägerlage 15 des Sensors ist bevorzugt durch ein flexibles, flächenhaftes Material gebildet. Die Erfassungsmittel 5, 6, 7 und 8 können dabei auf der Flachseite 14 der Trägerlage 15 angeordnet sein und somit bei angelegter Manschette einen direkten Kontakt mit der Hautoberfläche haben. Es ist aber auch möglich die angeordneten Erfassungsmittel mit einer Schutzschicht zu überziehen, wobei jedoch jeweils Abschnitte vorhanden sein müssen, in denen ein Aus- bzw. Zutritt der Wirkgröße zum bzw. vom Erfassungsmittel möglich ist. Gemäß einer vorteilhaften Weiterbildung kann die Trägerlage 15 bspw. durch ein transparentes bzw. semitransparentes Material gebildet sein, bspw. durch PET bzw. PANI. Diese Ausbildung hat den Vorteil, dass optisch wirkende Erfassungsmittel auch auf der, der Hautoberfläche abgewandten Flachseite der Trägerlage angeordnet werden können, ohne die optischen Wirksignale zu beeinträchtigen. Besonders bevorzugt ist eine Ausbildung, bei der die Erfassungsmittel 5, 6, 7 und 8 sowie das erste Kommunikationsmodul 10 auf die Flachseite 14 der Trägerlage 15 aufgedruckt werden. Insbesondere ist dies von Vorteil, wenn die Erfassungsmittel durch elektronische Bauteile aus organischem Halbleitermaterial gebildet werden.
Das Energieversorgungsmodul ist bspw. durch eine flächenhafte Trägerlage 19 gebildet, auf der das zweite Kommunikationsmodul 20, die Energiequelle 21, eine Auswertevorrichtung 22 sowie ein drittes Kommunikationsmodul 23 angeordnet sind. Das zweite Kommunikationsmodul 20 umfasst eine Sende- und/oder Empfangsvorrichtung 24 zur Aussendung bzw. zum Empfang einer, die Kommunikationsverbindung 4 bildenden, elektromagnetischen Welle ausgebildet ist. Über die Kommunikationsverbindung 4 ist das Energieversorgungsmodul 3 dazu ausgebildet, den Sensor 2 mit elektrischer Energie zu versorgen und von diesem die Messwerte der erfassten Vitalzeichen zu empfangen. Gegebenenfalls können auch noch Steuerungsdaten übertragen werden, bspw. das Aktivierungssignal an den Sensor und/oder das Triggersignal des Verschlussmittels.

Die Energiequelle 21 ist bevorzugt durch elektrochemische Energiespeicher wie Batterien und Akkumulatoren gebildet. Gemäß einer vorteilhaften Weiterbildung kann die Energiequelle auch durch eine Solarzelle, besonders bevorzugt eine organische Solarzelle, gebildet sein. Besonders bevorzugt ist eine Ausbildung, bei der der Energiespeicher in Dünnfilmtechnik hergestellt wird bspw. durch einen druckbaren Polymerakku.

Von besonderem Vorteil ist es, wenn der Energieverbrauch des Sensors, insbesondere der Erfassungsmittel, sehr gering ist, da in Messpausen der Energiespeicher durch Energieübertragung über die Haut wieder mit elektrischer Energie versorgt werden muss. Je weniger elektrische Energie zu übertragen ist, desto geringer ist die Belastung des menschlichen Körpers mit elektromagnetischen Feldern. Die International Radiation Protection Association (IRPA) hat diesbezüglich Grenzwerte für Feldstärken festgelegt, die auf den menschlichen Körper einwirken dürfen. Insbesondere wurde für die Spezifische Absorptionsrate (SAR) ein Grenzwert von 80mW/kg und für die Stromdichte (S) ein Grenzwert von 250mA/m² (rms) festgelegt.

Die erfindungsgemäße Vorrichtung ermöglicht in besonders vorteilhafter Weise eine zuverlässige Versorgung des Sensors mit elektrischer Energie, ohne den menschlichen Organismus einer übermäßigen Strahlungsbelastung auszusetzen, wobei jedoch eine ausreichend hohe Erfassungsrate von Vitalzeichen gegeben ist.

Zur Auswertung und Aufbereitung der erfassten Vitalzeichen kann das Energieversorgungsmodul auch eine Auswertevorrichtung 22 aufweisen, die zumindest eine Steuerungseinrichtung 25 und einen Speicher 26 umfasst. Beispielsweise sind zur Bestimmung des Sauerstoffgehalts des Bluts ein Vergleich und eine Bewertung von zwei, bei unterschiedlichen Spektralanteilen erfassten Messwerten erforderlich. Im Speicher 26 kann dazu bspw. eine Arbeitsanweisung hinterlegt sein, die von der Steuerungseinrichtung 25 geladen und ausgeführt wird, wobei als Ergebnis eine Maßzahl für den Sauerstoffgehalt des Bluts ermittelt wird. Selbstverständlich können noch weitere Arbeitsanweisungen hinterlegt sein. Im Hinblick auf eine Langzeitüberwachung können die so ermittelten Maßzahlen bspw. auch im Speicher 26 abgelegt werden, was den Vorteil hat, dass der Benutzer die ermittelten Daten nur unregelmäßig an die Datenanalyseeinrichtung übermitteln muss.

Zur Übermittlung der erfassten Vitalzeichen an die Datenanalyseeinrichtung weist das Energieversorgungsmodul 3 ein drittes Kommunikationsmodul 23 auf, das zur Herstellung einer Datenverbindung mit einer Datengegenstelle einer Datenanalyseeinrichtung ausgebildet ist. Diese Datenverbindung ist bevorzugt durch eine drahtlose, kurzreichweitige Verbindung gebildet, bspw. durch Bluetooth, RFID, IrDA. Das dritte Kommunikationsmodul 23 kann ebenfalls einen Speicher 27 aufweisen, was insbesondere dann von Vorteil ist, wenn bspw. keine Auswertevorrichtung 22 vorhanden ist, oder die Speicherkapazität der Auswertevorrichtung nicht ausreicht, um erfasste Vitalzeichen zwischen den einzelnen Synchronisationsvorgängen mit der Datenanalyseeinrichtung zwischenzuspeichern. Besonders bevorzugt ist der Speicher 26, 27 als nicht flüchtiges Speichermodul ausgeführt, da dann die erfassten Vitalzeichen auch bei einem Ausfall der Energiequelle 21 erhalten bleiben.

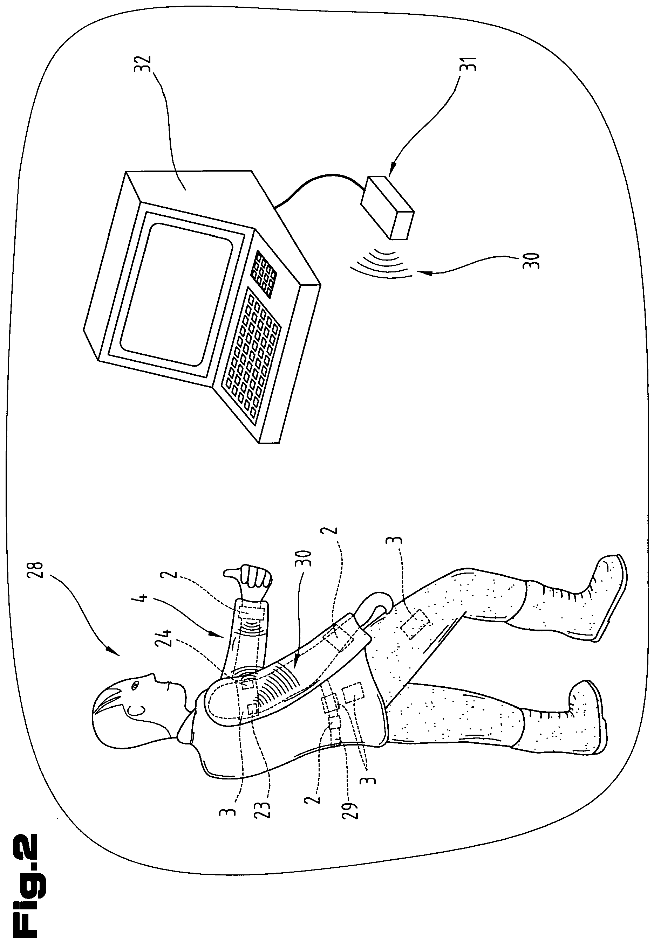

Fig. 2 zeigt einen Synchronisationsvorgang der erfassten Vitalzeichen mit einer Datenanalyseeinrichtung. Die erfindungsgemäße Erfassungsvorrichtung wird von der Person 28 getragen, wobei der Sensor 2 bspw. am Unterarm, insbesondere im Bereich des Handgelenks, als auch im Bereich des Oberkörpers getragen werden kann. Der Sensor 2 kann jedoch insbesondere an allen Körperpositionen angeordnet sein, wo eine Bestimmung von Vitalzeichen in vorteilhafter Weise einfach und zuverlässig möglich ist. Der Sensor 2 kann dabei als Manschette ausgebildet sein, die beim Anlegen an den Körperteil mittels eines Verschlusselements verschlossen und damit positioniert wird. Der Sensor kann jedoch auch mittels einer Haltevorrichtung 29 an einem Körperteil positioniert werden, wo die Ausbildung des Sensors als Manschette umständlich wäre, bzw. die Bewegungsfreiheit des Trägers zu stark einschränken würde.

Das Energieversorgungsmodul 3 kann in vorteilhafter Weise überall dort positioniert werden, wo es die Bewegungsfreiheit des Trägers 28 nicht oder nur unwesentlich einschränkt. Da die Kommunikationsverbindung 4 bevorzugt durch das Nahfeld der Haut gebildet ist, ist bei der Anordnung des Energieversorgungsmoduls 3 lediglich darauf zu achten, dass die Sende- und/ oder Empfangsvorrichtung 24 die abgestrahlte elektromagnetische Welle ausreichend gut an die Hautoberfläche ankoppeln kann.

Über das dritte Kommunikationsmodul 23 wird die Datenverbindung 30 zur Datengegenstelle 31 der Datenanalyseeinrichtung 32 aufgebaut. Die Datenanalyseeinrichtung kann bspw. durch ein weit verbreitetes Computersystem gebildet sein. Ein derartiges Computersystem ist zur Ausführung einer Programmlogik ausgebildet, um die über die Datenverbindung 31 übermittelten erfassten Vitalzeichen auszuwerten.

Die Datenverbindung 30 kann bspw. derart ausgebildet sein, dass die Synchronisation der erfassten Vitalzeichen automatisch startet, sobald sich der Benutzer 28 in der Nähe der Datengegenstelle 31 befindet. Insbesondere ist die Datenverbindung 30 jedoch derart ausgebildet, dass nur ein berechtigtes Energieversorgungsmodul 3, insbesondere ein berechtigtes drittes Kommunikationsmodul 23 eine Datenverbindung mit der Datengegenstelle 31 aufbauen kann. Somit wird eine unautorisierte und missbräuchliche Aufzeichnung von personenspezifischen Vitalzeichen durch unbefugte Dritte verhindert.

Die Ausführungsbeispiele zeigen mögliche Ausführungsvarianten der Erfassungsvorrichtung für Vitalzeichen, wobei an dieser Stelle bemerkt sei, dass die Erfindung nicht auf die speziell dargestellten Ausführungsvarianten derselben eingeschränkt ist, sondern vielmehr auch diverse Kombinationen der einzelnen Ausführungsvarianten untereinander möglich sind und diese Variationsmöglichkeit aufgrund der Lehre zum technischen Handeln durch gegenständliche Erfindung im Können des auf diesem technischen Gebiet tätigen Fachmannes liegt. Es sind also auch sämtliche denkbaren Ausführungsvarianten, die durch Kombinationen einzelner Details der dargestellten und beschriebenen Ausführungsvariante möglich sind, vom Schutzumfang mit umfasst.

Der Ordnung halber sei abschließend darauf hingewiesen, dass zum besseren Verständnis des Aufbaus der Erfassungsvorrichtung für Vitalzeichen, diese bzw. deren Bestandteile teilweise unmaßstäblich und/oder vergrößert und/oder verkleinert dargestellt wurden.

Die den eigenständigen erfinderischen Lösungen zugrunde liegende Aufgabe kann der Beschreibung entnommen werden.

Vor allem können die einzelnen in den Fig. 1 und 2 gezeigten Ausführungen den Gegenstand von eigenständigen, erfindungsgemäßen Lösungen bilden. Die diesbezüglichen, erfindungsgemäßen Aufgaben und Lösungen sind den Detailbeschreibungen dieser Figuren zu entnehmen.

### Bezugszeichenaufstellung

- 1: Erfassungsvorrichtung
- 2: Sensor
- 3: Energieversorgungsmodul
- 4: Kommunikationsverbindung
- 5: Erfassungsmittel für den Sauerstoffgehalt des Bluts

- 6: Erfassungsmittel für die Körpertemperatur
- 7: Erfassungsmittel für die Hautfeuchte
- 8: Erfassungsmittel für die Leitfähigkeit der Haut
- 10: erstes Kommunikationsmodul

- 11: Verschlusselement
- 12: Elektromagnetischer Strahlungsdetektor
- 13: Quelle für elektromagnetische Strahlung
- 14: Flachseite
- 15: Trägerlage

- 16: Sende- und/oder Empfangsvorrichtung
- 17: Energiespeicher
- 18: Elektrische Verbindungsleitung
- 19: Trägerlage
- 20: zweites Kommunikationsmodul

- 21: Energiequelle
- 22: Auswertevorrichtung
- 23: drittes Kommunikationsmodul
- 24: Sende- und/oder Empfangsvorrichtung
- 25: Steuerungseinrichtung

- 26: Speicher
- 27: Speicher
- 28: Benutzer, Person, Träger
- 29: Haltevorrichtung
- 30: Datenverbindung

- 31: Datengegenstelle
- 32: Datenanalyseeinrichtung

## Patentansprüche

1. Erfassungsvorrichtung (1) für Vitalzeichen umfassend einen Sensor (2) und ein Energieversorgungsmodul (3), wobei der Sensor (2) als Dünnfilmsensor und elastisch rückstellbar verformbare Manschette ausgebildet ist und zumindest ein Erfassungsmittel (5, 6, 7, 8) aufweist, das zur Erfassung eines Vitalzeichens aus der Gruppe umfassend Sauerstoffgehalt des Bluts, Körpertemperatur, Hautfeuchtigkeit, Leitfähigkeit der Haut, Puls ausgebildet ist und ein erstes (10) Kommunikationsmodul umfasst, wobei das Energieversorgungsmodul (3) ein zweites Kommunikationsmodul (20) und eine Energiequelle (21) aufweist und wobei zwischen dem ersten (10) und zweiten Kommunikationsmodul (20) eine drahtlose Kommunikationsverbindung (4) besteht, **dadurch gekennzeichnet, dass** die Kommunikationsverbindung (4) durch das Nahfeld der Haut gebildet ist und wobei die Kommunikationsverbindung (4) zur Übertragung von Energie und Messwerten bzw. Messdaten ausgebildet ist und wobei ferner die Manschette ein Verschlusselement (11) aufweist, das zur Abgabe eines Triggersignals ausgebildet ist, wobei das Triggersignal zur Aktivierung der Erfassungsvorrichtung (1) ausgebildet ist, welche Erfassungsvorrichtung (1) hernach periodische Vitalsignale erfasst.

2. Erfassungsvorrichtung nach Anspruch 1, **dadurch gekennzeichnet, dass** das Erfassungsmittel für den Sauerstoffgehalt des Bluts (5) durch zumindest eine Quelle für elektromagnetische Strahlung (13) und einen elektromagnetischen Strahlungsdetektor (12) gebildet ist.

3. Erfassungsvorrichtung nach einem der Ansprüche 1 oder 2, **dadurch gekennzeichnet, dass** das Erfassungsmittel (5, 6, 7, 8) durch organische und/oder anorganische Halbleiterbauteile gebildet ist.

4. Erfassungsvorrichtung nach einem der Ansprüche 1 bis 3, **dadurch gekennzeichnet, dass** der Sensor (2) durch eine flächenhafte Trägerlage (15) gebildet ist, auf der das Erfassungsmittel (5, 6, 7, 8) angeordnet ist.

5. Erfassungsvorrichtung nach Anspruch 4, **dadurch gekennzeichnet, dass** die Trägerlage (15) transparent bzw. semitransparent ausgebildet ist.

6. Erfassungsvorrichtung nach einem der Ansprüche 4 oder 5, **dadurch gekennzeichnet, dass** das Erfassungsmittel (5, 6, 7, 8) auf einer Flachseite (14) der Trägerlage (15) aufgedruckt ist.

7. Erfassungsvorrichtung nach einem der Ansprüche 1 bis 6, **dadurch gekennzeichnet, dass** das erste Kommunikationsmodul (10) einen Speicher für elektrische Energie (17) aufweist.

8. Erfassungsvorrichtung nach einem der Ansprüche 1 bis 7, **dadurch gekennzeichnet, dass** das erste (10) und zweite (20) Kommunikationsmodul eine Sende- und/oder Empfangsvorrichtung (16) für elektromagnetische Strahlung aufweist.

9. Erfassungsvorrichtung nach einem der Ansprüche 1 bis 8, **dadurch gekennzeichnet, dass** die Energiequelle (17) durch ein Element aus der Gruppe umfassend chemisches Element, anorganische bzw. organische Solarzelle, gebildet ist.

10. Erfassungsvorrichtung nach einem der Ansprüche 1 bis 9, **dadurch gekennzeichnet, dass** das Energieversorgungsmodul (3) eine Auswertevorrichtung (22) aufweist.

11. Erfassungsvorrichtung nach Anspruch 10, **dadurch gekennzeichnet, dass** die Auswertevorrichtung (22) eine Steuerungseinrichtung (25) und einen Speicher (26) umfasst.

12. Erfassungsvorrichtung nach einem der Ansprüche 1 bis 11, **dadurch gekennzeichnet, dass** das Energieversorgungsmodul (3) ein drittes Kommunikationsmodul (23) aufweist.

13. Erfassungsvorrichtung nach Anspruch 12, **dadurch gekennzeichnet, dass** das dritte Kommunikationsmodul (23) zur Herstellung einer Datenverbindung (30) mit einer Datengegenstelle (31) einer Datenanalyseeinrichtung (32) ausgebildet ist.

14. Erfassungsvorrichtung nach Anspruch 12 oder 13, **dadurch gekennzeichnet, dass** das dritte Kommunikationsmodul (23) ein Speichermodul aufweist.

15. Erfassungsvorrichtung nach einem der Ansprüche 1 bis 14, **dadurch gekennzeichnet, dass** das Energieversorgungsmodul (3) in bzw. an der Kleidung des Benutzers angeordnet sein kann.

16. Erfassungsvorrichtung nach einem der Ansprüche 1 bis 15, **dadurch gekennzeichnet, dass** das Energieversorgungsmodul (3) zur Aktivierung des Sensors (2) ausgebildet ist.

## Claims

1. A detection device (1) for vital signs, comprising a sensor (2) and an energy supply module (3), wherein the sensor (2) is designed as a thin film sensor and elastically resettable cuff and comprises at least one detection means (5, 6, 7, 8) designed to detect a vital sign from the group comprising oxygen content of the blood, body temperature, skin moisture, conductivity of the skin, pulse, and a first (10) communication module, wherein the energy supply module (3) has a second communication module (20) and an energy source (21), and wherein a wireless communication link (4) exists between the first (10) and second communication modules (20), **characterized in that** the first communication link (4) is formed by the near field of the skin, and wherein the communication link (4) is configured for transmitting energy and measurement values and/or measurement data and wherein, furthermore, the cuff has a closure element (11) designed for emitting a trigger signal, wherein the trigger signal is configured for activating the detection device (1), which detection device (1) subsequently detects periodic vital signals.

2. The detection device according to claim 1, **characterized in that** the means (5) for detecting the oxygen content of the blood is provided in the form of at least one source of electromagnetic radiation (13) and an electromagnetic radiation detector (12).

3. The detection device according to any one of claims 1 or 2, **characterized in that** the detection means (5, 6, 7, 8) is provided in the form of organic and/or inorganic semiconductor components.

4. The detection device according to any one of claims 1 to 3, **characterized in that** the sensor (2) is provided in the form of a flat support layer (15) on which the detection means (5, 6, 7, 8) is disposed.

5. The detection device according to claim 4, **characterized in that** the support layer (15) is of a transparent or semi-transparent design.

6. The detection device according to any one of claims 4 or 5, **characterized in that** the detection means (5, 6, 7, 8) is printed onto a flat face (14) of the support layer (15).

7. The detection device according to anyone of claims 1 to 6, **characterized in that** the first communication module (10) has a storage for electrical power (17).

8. The detection device according to any one of claims 1 to 7, **characterized in that** the first (10) and second (20) communication modules have a transmitter and/or receiver device (16) for electromagnetic radiation.

9. The detection device according to any one of claims 1 to 8, **characterized in that** the power source (17) is provided in the form of an element from the group comprising chemical elements, an inorganic or organic solar cell.

10. The detection device according to any one of claims 1 to 9, **characterized in that** the power supply module (3) has an evaluation device (22).

11. The detection device according to claim 10, **characterized in that** the evaluation device (22) comprises a control unit (25) and a memory (26).

12. The detection device according to any one of claims 1 to 11, **characterized in that** the power supply module (3) has a third communication module (23).

13. The detection device according to claim 12, **characterized in that** the third communication module (23) is configured to establish a data link (30) to a data distant end (31) of a data analysis unit (32).

14. The detection device according to claim 12 or 13, **characterized in that** the third communication module (23) has a memory module.

15. The detection device according to any one of claims 1 to 14, **characterized in that** the power supply module (3) may be disposed in or on the user's clothing.

16. The detection device according to any one of claims 1 to 15, **characterized in that** the power supply module (3) is configured to activate the sensor (2).

## Revendications

1. Dispositif de mesure (1) de signes vitaux comprenant un capteur (2) et un module d'alimentation en énergie (3), le capteur (2) étant conçu comme un capteur à film mince et comme une manchette déformable de manière élastique et réversible, et comprenant au moins un moyen de mesure (5, 6, 7, 8), qui est conçu pour la mesure d'un signe vital sélectionné dans le groupe comprenant la concentration en oxygène du sang, la température corporelle, l'humidité de la peau, la conductivité de la peau, le pouls, et comprenant un premier module de communication (10), le module d'alimentation en énergie (3) comprenant un deuxième module de communication (20) et une source d'énergie (21) et, entre le premier (10) et le deuxième module de communication (20), une liaison de communication sans fil (4) étant établie, **caractérisé en ce que** la liaison de communication (4) est constituée par le champ proche de la peau et la liaison de communication (4) étant conçue pour la transmission d'énergie et de valeurs de mesure ou de données de mesure et la manchette comprenant en outre un élément de fermeture (11) qui est conçu pour la production d'un signal de déclenchement, le signal de déclenchement étant conçu pour l'activation du dispositif de mesure (1), ce dispositif de mesure (1) mesurant alors des signes vitaux périodiques.

2. Dispositif de mesure selon la revendication 1, **caractérisé en ce que** le moyen de mesure pour la concentration en oxygène du sang (5) est constitué d'au moins une source de rayonnement électromagnétique (13) et d'un détecteur de rayonnement électromagnétique (12).

3. Dispositif de mesure selon l'une des revendications 1 ou 2, **caractérisé en ce que** le moyen de mesure (5, 6, 7, 8) est constitué de composants semi-conducteurs organiques et/ou inorganiques.

4. Dispositif de mesure selon l'une des revendications 1 à 3, **caractérisé en ce que** le capteur (2) est constitué d'une couche de support plane (15) sur laquelle est disposé le moyen de mesure (5, 6, 7, 8).

5. Dispositif de mesure selon la revendication 4, **caractérisé en ce que** la couche de support (15) est conçu de façon à être transparente ou semi-transparente.

6. Dispositif de mesure selon l'une des revendications 4 ou 5, **caractérisé en ce que** le moyen de mesure (5, 6, 7, 8) est imprimé sur un côté plat (14) de la couche de support (15).

7. Dispositif de mesure selon l'une des revendications 1 à 6, **caractérisé en ce que** le premier module de communication (10) comprend un accumulateur d'énergie électrique (17).

8. Dispositif de mesure selon l'une des revendications 1 à 7, **caractérisé en ce que** le premier (10) et le deuxième (20) module de communication comprend un dispositif d'émission et/ou de réception (16) pour un rayonnement électromagnétique.

9. Dispositif de mesure selon l'une des revendications 1 à 8, **caractérisé en ce que** la source d'énergie (17) est constituée d'un élément sélectionné dans le groupe comprenant un élément chimique, une cellule photovoltaïque inorganique ou organique.

10. Dispositif de mesure selon l'une des revendications 1 à 9, **caractérisé en ce que** le module d'alimentation en énergie (3) comprend un dispositif d'analyse (22).

11. Dispositif de mesure selon la revendication 10, **caractérisé en ce que** le dispositif d'analyse (22) comprend un dispositif de commande (25) et une mémoire (26).

12. Dispositif de mesure selon l'une des revendications 1 à 11, **caractérisé en ce que** le module d'alimentation en énergie (3) comprend un troisième module de communication (23).

13. Dispositif de mesure selon la revendication 12, **caractérisé en ce que** le troisième module de communication (23) est conçu pour l'établissement d'une liaison de données (30) avec un terminal de données (31) d'un dispositif d'analyse de données (32).

14. Dispositif de mesure selon la revendication 12 ou 13, **caractérisé en ce que** le troisième module de communication (23) comprend un module de mémoire.

15. Dispositif de mesure selon l'une des revendications 1 à 14, **caractérisé en ce que** le module d'alimentation en énergie (3) peut être disposé dans ou sur les vêtements de l'utilisateur.

16. Dispositif de mesure selon l'une des revendications 1 à 15, **caractérisé en ce que** le module d'alimentation en énergie (3) est conçu pour activer le capteur (2).
